# EUROPEAN PATENT SPECIFICATION

(11) **EP 2 883 571 B1**
(45) Date of publication and mention of the grant of the patent: **12.09.2018**
(21) Application number: 13197243.2
(22) Date of filing: 13.12.2013
(51) Int. Cl.: A61Q 5/08, A61K 8/43, A61K 8/31, A61K 8/19, A61K 8/25, A61Q 5/10

(54) **Hair lightening/bleaching composition**
Haaraufhellungs-/-bleichzusammensetzung
Composition de blanchiment/d'éclaircissement des cheveux

(43) Date of publication of application: 17.06.2015
(73) Proprietor: Kao Corporation, Chuo-ku Tokyo 103-8210 (JP)
(72) Inventor: Feier-Iova, Ovidiu, 64283 Darmstadt (DE)
(74) Representative: Hoffmann Eitle

(56) References cited:
- EP-A1- 0 560 088
- EP-A1- 0 890 355
- EP-A1- 2 471 502
- DE-U1- 29 722 990
- FR-A1- 2 940 076

## Description

Present invention relates to a hair lightening/bleaching composition comprising two alkaline salts.

There are two main types of bleaching/lightening compositions which have been known for many years. The first is an anhydrous composition (usually powder) which comprises, as a rule, per salts and alkalizing agents and which is applied onto hair after mixing with an aqueous acidic composition comprising at least one oxidizing agent. Such lightening powders usually deliver satisfactory lightening but hair damage is very high and they are not compatible with standard hair dyestuffs. This has the disadvantage that two processes are needed to lighten and tone the hair. Additionally and importantly, they are not mild enough so that scalp/skin irritations are often observed so that cautions must be taken when applying directly onto skin in order to bleach hair at growth area.

The second type of lightener is usually an aqueous alkaline cream emulsion composition which is free of per salts and which is applied onto hair after mixing with an aqueous acidic composition comprising at least one oxidizing agent. Such lighteners are compatible with standard hair dyestuffs allowing simultaneous lightening and toning but do not deliver satisfactory lightening, especially on hair that has already been colored with oxidative dyes and therefore hair comprising parts colored previously with an oxidative dyeing composition and uncoloured parts cannot always be bleached homogeneously. In other words, such lightening technology only provides selective bleaching for natural and previously oxidatively coloured hair so that homogeneous colour appearance of such hair is not observed.

DE 29722990 relates to bleaching agents for lightening the hair, comprising at least one water-soluble guanidine salt. The agent may be provided as a bleaching powder and may further comprise peroxide salts such as persulfates.

EP 2 471 502 relates to substantially water free bleaching and/or highlighting composition for keratin fibres comprising at least one compound with bleaching and/or highlighting effect and a polyhydroxy carboxylic acid as a chelating agent.

FR 2 940 076 describes a procedure for lightening keratin fibers, preferably human keratin fibers such as hair, which comprises applying an anhydrous cosmetic composition (A) comprising one or more fatty substance and one or more surfactants, a cosmetic composition (B) comprising one or more organic amine salts having a negative logarithm of base dissociation constant (pKb) value of less than 12 at 25°C; and a composition (C) comprising one or more oxidizing agents, on the keratin fibers.

In view of the above, aim of the present invention is to provide a hair lightening and/or bleaching composition which provides effective lightening of natural and previously coloured hair but causes less damage to the hair and milder to scalp than the presently available bleach compositions.

Present inventors have unexpectedly found out that an anhydrous composition comprising two alkaline salts suspended in an oil provide effective lightening with less damaging effect when used in combination with an alkalizing agent and after mixing with an aqueous acidic composition comprising at least one oxidizing agent.

Accordingly, the first object of the present invention is an anhydrous composition for lightening and/or bleaching hair comprising at least one alkalizing agent, at least one guanidine salt, at least one second alkaline salt selected from salts of carbonates, bicarbonates, phosphate and hydrogenphosphates with a cation selected from sodium, potassium or ammonium and one or more oil liquid at 20°C, the oil concentration being in the range of 20 to 60% by weight calculated to the total composition.

The compositions preferably do not comprise the known per salts at a concentration of higher than 10% by weight and preferably 5% by weight and most preferably free of per salts, all values are calculated to the total of the composition.

Second object of the present invention is the use of the composition for lightening and/or bleaching human hair.

Third object of the present invention is a process for lightening and/or bleaching human hair wherein the composition is mixed with an aqueous composition which has an acidic pH and comprises at least one oxidizing agent, preferably hydrogen peroxide and is applied onto hair and after being left on the hair for 1 to 45 min is rinsed off from hair, and hair is optionally dried.

The fourth object of the present invention is kit for hair comprising two or more compositions wherein one of the compositions is the composition of the present invention and the other composition is an aqueous composition comprising at least one oxidizing agent having an acidic pH.

For example, EP 890 355 A1 discloses hair dyeing compositions comprising guanidium salt such as guanidium carbonate. The document does not disclose anything on combining the guanidium salt with another alkaline salt and/or with an alkalizing agent.

EP 642 783 discloses oxidative hair dyeing compositions comprising ammonium salts. Among the suitable salts ammonium carbonate and ammonium bicarbonate are mentioned. However, the document fails to disclose any combination with guanidium salts.

Furthermore, EP 560088 discloses powder bleaching composition comprising per salts and sodium carbonate. The document fails to disclose anhydrous bleaching composition comprising two alkaline salts and does not disclose at all any guanidium salts.

The composition of the present invention comprises at least one guanidine salt. Suitable ones are guanidine carbonate, guanidine hydrochloride and guanidine phosphate. Particularly preferred is guanidine carbonate.

The concentration of at least one guanidium salt is in the range of 5 to 60%, preferably 10 to 55%, more preferably 15 to 50% and most preferably 15 to 40% by weight calculated to the total of the composition.

The composition of the present invention comprises at least one alkaline salt selected from salts of carbonate, bicarbonate, phosphates and hydrogen phosphates with a cation selected from sodium, potassium or ammonium. Suitable salts are sodium carbonate, sodium bicarbonate, trisodium phosphate, disodium hydrogen phosphate, sodium dihydrogen phosphate, potassium carbonate, potassium bicarbonate, tripotassium phosphate, dipotassium hydrogen phosphate, potassium dihydrogen phosphate, ammonium carbonate, ammonium bicarbonate, triammonium phosphate, diammonium hydrogen phosphate and ammonium dihydrogen phosphate. Preferred are sodium bicarbonate, sodium carbonate, potassium bicarbonate, potassium carbonate, ammonium carbonate and ammonium bicarbonate. Particularly preferred are sodium bicarbonate, potassium bicarbonate and ammonium bicarbonate. Ammonium bicarbonate is the especially preferred alkaline salt.

The concentration of at least one alkaline salt is in the range of in the range of 5 to 60%, preferably 10 to 55%, more preferably 15 to 50% and most preferably 15 to 40% by weight calculated to the total of the composition.

It has also been found out that the weight ratio between the two salts may play a role in effective lightening of artificially colored hair. The weight ratio between guanidine salt and the second alkaline salt is in the range of 10:1 to 1:5, preferably 5:1 to 1:2, more preferably 3:1 to 1:2 and most preferably 2:1 to 1:1.

The compositions of the present invention comprise at least one alkalizing agent. Suitable and preferred alkalizing agent is sodium metasilicate and is comprised in the composition in the range of 1 to 20%, preferably 2 to 15% more preferably 2.5 to 12.5 and most preferably 5 to 10% by weight calculated to the total of the composition.

The composition comprises one or more oil liquid at 20°C. The total concentration of oil is in the range of in the range of 20 to 60%, preferably 25 to 55%, more preferably 25 to 50% and most preferably 30 to 45% by weight calculated to the total of the composition.

One or more oil may be selected from synthetic and natural oils. Synthetic oils are silicones especially those of nonvolatile ones such as dimethicones with viscosity of 50 to 350 cSt measured by capillary viscosimeter and at 20°C, fatty acid fatty alcohol esters according to the general structure

R₅₀C(O)R₅₁

wherein R₅₀ is a straight or branched, saturated or unsaturated alkyl with 11 to 21 C atomes and R₅₁ is a straight or branched, saturated or unsaturated alkyl with 1 to 22 C atomes such as behenyl behenate, behenyl isostearte, butyl stearate, butyl oleate, butyl myristate,butyloctyl oleate,cetyl palmitate, cetyl myristate, cetyl oleate, cetyl caprylate, cetyl caprate, decyl oleate, decyl cocoate, decyl isostearate, ethylhexyl myristate, ethyl hexyl laurate, ethyl hexyl oleate, ethyl isostearte, ethyl laurate, ethyl linoleate, ethyl myristate, ethyl oleate, ethyl palmitate, ethylricinoleate, ethyl stearate, hexyl isostearet, hexyl laurate, hexyl myristate, hexyl stearate, hexyl decyl oleate, isobutyl laurate, isobutyl myristate, isobutyl palmitate, isobutyl stearate, isocetyl behenate, isobutyl laurate, isobutyl oleate, isobutyl stearate, isobutyl cocoate, isohexyl caprate, isopropyl palmitate, isopropyl stearate, isopropyl behenate, isopropyl laurate, isopropyl oleate, isopropyl ricinoleate and isopropyl palmitate, and fatty alcohol ethers according to general structure

R₃₀OR₄₀

wherein R₃₀ and R₄₀ are same or different, straight or branched, saturated or unsaturated alkyl with 8 to 22 C atoms such as dicetyl ether, dimyristyl ether, dicyprylyl ether and dodecyl ether.

Natural oils are mineral oil and plant derived triglycerides such as ricinus oil, soja oil avocado oil, olive oil, almond oil, peach oil, passiflora oil, black cumin oil, borage oils, evening primrose oil, grape seed oil, hempseed oil, kukui nut oil, rosehip oil, safflower oil, walnut oil and wheat germ oil.

The most preferred oil is mineral oil.

The composition of the present invention may comprise inert carrier material such as starch, diatomaceous earth, pyrogenic silicium dioxide.

Further, the composition comprises thickening polymers, preferably selected from the group consisting of cellulose polymer compounds, alginate, polysaccarides and acrylic acid polymers, preferably methyl cellulose compounds, ethyl cellulose compounds, hydroxyethylcellulose compounds, methylhydroxyethylcellulose compounds, methylhydroxypropylcellulose compounds, carboxymethyl cellulose compounds, alginic acids, sodium alginates, ammonium alginates, calcium alginates, gum arabic, guar gum or xanthan gum, or acrylic acid polymers with molecular weights from about 1,250,000 to 4,000,000, alone or in combination with each other. The polymers are used in a total amount of 0.1 to 15 %, preferably from 0.2 to 10 %, and more preferably in an amount of from 0.5 to 7.5% by weight, calculated to total of the composition.

The composition can also comprise cationic polymers as conditioning and/or thickening agents. Preferred ones are those cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Furthermore, other cationic polymers known with their CTFA category name Polyquaternium are also suitable as conditioners and/or thickener. Typical examples of those Polyquaternium 1, Polyquaternium 2, Polyquaternium 4, Polyquaternium 5, Polyquaternium 6, Polyquaternium 7, Polyquaternium 8, Polyquaternium 9, Polyquaternium 11, Polyquaternium 12, Polyquaternium 13, Polyquaternium 14, Polyquaternium 15, Polyquaternium 16, Polyquaternium 17, Polyquaternium 18, Polyquaternium 19, Polyquaternium 20, Polyquaternium 22, Polyquaternium 24, Polyquaternium 27, Polyquaternium 28, Polyquaternium 29, Polyquaternium 30, Polyquaternium 31, Polyquaternium 32, Polyquaternium 33, Polyquaternium 34, Polyquaternium 35 and Polyquaternium 36, Polyquaternium-37, Polyquaternium 39, Polyquaternium 42, Polyquaternium 43, Polyquaternium 44, Polyquaternium 45, Polyquaternium 46, Polyquaternium 47, Polyquaternium 48, Polyquaternium-49, Polyquaternium 50, Polyquaternium 51, Polyquaternium 52, Polyquaternium 53, Polyquaternium 54, Polyquaternium 55, Polyquaternium 56, Polyquaternium 57, Polyquaternium 58, Polyquaternium 59, Polyquaternium 60, Polyquaternium 61, Polyquaternium 62, Polyquaternium 63, Polyquaternium 64, Polyquaternium 65, Polyquaternium 66, Polyquaternium 67, Polyquaternium 68, Polyquaternium 69, Polyquaternium-70, Polyquaternium 71, Polyquaternium 72, Polyquaternium 73, Polyquaternium 74, Polyquaternium 75, Polyquaternium 76, Polyquaternium 77, Polyquaternium 78, Polyquaternium-79, Polyquaternium 80, Polyquaternium 81, Polyquaternium 82, Polyquaternium 83, Polyquaternium 84, Polyquaternium 85, Polyquaternium 86 and Polyquaternium 87.

Typical concentration range for any of the cationic conditioners mentioned above can be 0.1 - 7.5% by weight, preferably 0.3 - 5% by weight and more preferably 0.5 - 2.5% by weight, calculated to total of the composition.

In another form of the present invention, the composition may be mixed into the oxidative dyeing composition prior to application onto hair. In this form of application of the present invention, the alkalizing agent may be comprised in the oxidative dyeing composition and therefore, in such a case, the composition may be free of alkalizing agent.

Thus, in another preferred form of carrying out the present invention is a process wherein an anhydrous composition comprising at least one guanidine salt, at least one second alkaline salt selected from salts of carbonates, bicarbonates, phosphate and hydrogenphosphates with a cation selected from sodium, potassium or ammonium, an one or more oil is mixed with two aqueous compositions wherein the first composition (Dyeing Composition) is an aqueous composition comprising at least one alkalizing agent and one or more hair dyes and the second composition (Oxidizing Composition) is an aqueous composition comprising at least one oxidizing agent and has an acidic pH, prior to application onto hair and is left on the hair for 1 to 45 min and is rinsed off from hair and hair is optionally dried.

The above disclosure on the anhydrous composition is also valid for the anhydrous composition used in the above process except for the alkalizing agent.

The aqueous Dyeing Composition of the above process comprises at least one alkalizing agent. Suitable ones are ammonia and compounds according to the general formula

R₁R₂R₃N

wherein R₁, R₂ and R₃ are same or different H, C₁ - C₆ alkyl, C₁ - C₆ monohydroxyalkyl, branched C₃ - C₆ monohydroxyalkyl or C₂ - C₆ polyhydroxyalkyl with the condition that at least one of R₁, R₂ and R₃ is not H and preferably a mono or polyhydroxyalkyl. Further preferably R₁, R₂ and R₃ are same or different H, C₁ - C₄ alkyl, C₁ - C₄ monohydroxyalkyl, branched C₃ - C₄ monohydroxyalkyl or C₂ - C₄ polyhydroxyalkyl with the condition that at least one of R₁, R₂ and R₃ is not H and preferably a mono or polyhydroxyalkyl.

Suitable alkalizing agents are ammonia, monoethanolamine, diethanolamine, triethanolamine, monoethanol methylamine, monoethanoldimethylamine, di-ethanolmethylamine, monoethanolethylamine, monoethanoldiethylamine, diethanolethylamine, monoethanolpropylamine, monoethanoldipropylamine, diethanolpropylamine, monoethanolbutylamine, diethanolbutylamine and amino methyl propanol and their mixtures.

Preferred are ammonia, monoethanolamine, diethanolamine, triethanolamine and amino methyl propanol and their mixtures. The most preferred are ammonia, monoethanolamine and amino methyl propanol and their mixtures.

The concentration of total alkalizing agent in the compositions varies between 1 and 35%, preferably 1 and 30, more preferably 2.5 and 25 and most preferably 2.5 to 20% by weight calculated to the total the dyeing composition.

The aqueous composition comprises at least one hair dye. Suitable ones are oxidative dye precursor, coupling substance and direct dyes.

Suitable oxidative dyestuffs precursors are tetraaminopyrimidines, in particular 2,4,5,6-tetraaminopyrimidine and the lower alkyl derivatives thereof; suitable triaminohydroxypyrimidines are, for example 4-hydroxy-2,5,6-triaminopyrimidine, 2-hydroxy-4,5,6-triaminopyrimidine and 5-hydroxy-2,4,6-triaminopyrimidine; suitable mono- and diamino dihydroxypyrimidines are, for example, 2,6-dihydroxy-4,5-diaminopyrimidine, 2,4-diamino-6-hydroxy-pyrimidine or 4,6-dihydroxy-2,5-diaminopyrimidine or the water-soluble salts thereof, aminophenol derivatives such as 4-aminophenol, 4-amino-3-methylphenol, 2-chloro-4-aminophenol, 2,6-dichloro-4-aminophenol, 2,4-diamino-phenol, 2,6-dibromo-4-aminophenol and/or 2-aminophenol and water-soluble salts thereof, furthermore, phenylenedimanine derivatives such as 2,5-diamino-toluene, 2-n-propyl or 2-ethyl-p-phenylene-diamine, 2,6-dimethyl-p-phenylenediamine, 2-(2,5-diaminophenyl) ethanol, 1-amino -4-bis-(2'-hydroxy-ethyl)aminobenzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene and/or 1-amino-4-β-hydroxypropyl aminobenzene or the water-soluble salts thereof, pyrazole derivatives such as 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 1-methyl-4,5-diaminopyrazole, 1-methylethyl-4,5-diaminopyrazole, 1-phenylmethyl-4,5-diaminopyrazole, 1-methyl-4,5-diaminopyrazole, 1-(4-methylphenyl)methyl-4,5-diaminopyrazole, 1-methyl-3-phenyl-4,5-diaminopyrazole and the water-soluble salts. The use of the above mentioned oxidative dye precursors as mixture is also customary in hair coloring area.

The dyeing composition comprises preferably at least one coupling substance, which can be selected from resorcinol, 2-methyl resorcinol, 4-chlororesorcinol, 2-amino-4-chlorophenol, 5-amino-4-methoxy-2-methylphenol, 3-amino-phenol, 1-methyl-2-hydroxy-4-aminobenzene, 3-N,N-dimethyl aminophenol, 2.6-dihydroxy-3.5-dimethoxypyridine, 5-amino-3-methylphenol, 6-amino-3-methylphenol, 3-amino-2-methylamino-6-methoxypyridine, 2-amino-3-hydroxy-pyridine, 2-dimethyl-amino-5-aminopyridine, 2,6-diaminopyridine, 1,3-diamino- benzene, 1-amino-3-(2'-hydroxyethylamino)benzene, 1-amino-3-[bis(2'-hydroxy-ethyl) amino]benzene, α-naphthol, 4,6-dichlororesorcinol, 1,3-diamino-toluene, 1-hydroxy naphthalene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1.2-methyldioxy benzene, 2,4-diamino-3-chlorophenol, 5-amino-2-methoxyphenol and/or 1-methoxy-2-amino-4-(2'-hydroxyethyl amino)benzene or the water-soluble salts thereof.

The total concentration of the oxidation dyestuff precursors and/or their water soluble salts and the coupling substances customarily ranges between 0.01% and 10%, preferably 0.05% and 8 %, in particular 0.1% to 6% by weight, calculated to the total hair dyeing composition.

The weight proportion of the named oxidative dye precursors to the coupling substances ranges between about 1 : 8 to 8 : 1, preferably about 1 : 5 to 5 : 1, in particular 1 : 2 to 2 : 1.

The aqueous dyeing composition comprises direct dyes of neutral, cationic and anionic character. Some examples to suitable cationic dyes are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 51, Basic Red 76, Basic Violet 1, Basic Violet 2, Basic Violet 3, Basic Violet 10, Basic Violet 14 and Basic Yellow 57. According to the invention, suitable cationic dyestuffs are in principal those any available on the market for cosmetic hair colouring applications. For this purpose, special reference is made to the PCT application WO 95/15144 of Ciba-Geigy AG. The content of the PCT application WO 95/15144 is by reference incorporated here.

Examples to suitable direct acting anionic dyes are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Some examples to those suitable neutral dyes (HC dyes), so called nitro dyes, are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No. 10, HC Yellow No.11, HC Yellow No.12, HC Yellow No.13, HC Yellow No.14, HC Yellow No.15, 2-Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs can also be used alone or in combination with synthetic direct-acting dyestuffs, for example henna (red or black), alkanna root, laccaic acid, indigo, logwood powder, madder root and rhubarb powder, etc.

According to the invention, the dyeing composition comprises direct hair dyes at a total concentration of 0.01 to 10%, preferably 0.05 to 7.5%, more preferably 0.1 to 5% by weight calculated to the total the dyeing composition.

The aqueous dyeing compositions may be in the form of solutions, dispersions, gels and emulsions. Emulsions are the most preferred from.

The aqueous emulsion composition preferably comprises one or more fatty alcohol of the general formula

R₄-OH

wherein R₄ is a linear or branched, saturated or unsaturated alkyl chain with 12 to 22 C atoms and at least one emulsifier selected from anionic, non-ionic, cationic and amphoteric surfactants.

Suitable fatty alcohols are myristyl alcohol, cetyl alcohol, stearyl alcohol, oleyl alcohol, octyldodecanol. and behenyl alcohol and their mixtures. Preferred is the mixture of cetyl and stearyl alcohol also known as cetearyl alcohol.

The concentration of one or more fatty alcohols is in the range of 1 to 25%, preferably 2.5 to 20%, more preferably 5 to 15% and most preferably 5 to 12% by weight calculated to total of the dyeing composition.

The aqueous emulsion dyeing composition comprises at least one emulsifier selected from anionic, non-ionic, cationic and amphoteric surfactants. Preferred emulsifying surfactants are anionic, non-ionic and cationic ones and especially preferred are the mixture of anionic and non-ionic surfactants and mixture of cationic and non-ionic surfactants at any ratio. Preferred mixing ratio for the anionic - non-ionic emulsifying surfactant mixture and cationic - non-ionic emulsifying surfactant mixture is in the range of 5:1 to 1:5, more preferably 3:1 to 1:3 and especially 1:1, by weight. It should be noted that incompatibilities can arise when anionic and cationic surfactants are used as the mixture emulsifier which should be taken into account when selecting such a combinations.

In principal any anionic surfactant is suitable within the meaning of the present invention. Nonlimiting examples are anionic surfactants of the sulfate, sulfonate, carboxylate and alkyl phosphate type, especially, of course, those customarily used as emulsifiers, for example, the known d₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates and their salts.

Further suitable surfactants of the carboxylate type are alkyl polyether carboxylic acids and the salts thereof of the formula

R₅-(OC₂H₄)ₙ-O-CH₂COOX,

wherein R₅ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 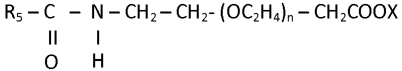 wherein R₅ and X have the above meanings, and n is in particular a number from 1 to 10, preferably 2.5 to 5.

Among the anionic surfactants most preferred are alkyl sulfates and/or alkyl ether sulfates and among them sodium lauryl or laureth sulfates and their mixtures are most preferred.

Suitable non-ionic surfactants are alkyl polyglucosides of the general formula

R₆-O-(R₇O)ₙO-Zₓ

wherein R₆ is an alkyl group with 8 to 18 carbon atoms, R₇ is an ethylene or propylene group, Z is a saccharide group with 5 to 6 carbon atoms, n is a number from 0 to 10 and x is a number between 1 and 5. Examples are decyl polyglucoside and cocoyl polyglucoside, both beeing commercially available.

Further nonionic surfactant components are, for example, long-chain fatty acid mono- and dialkanolamides, such as coco fatty acid monoethanolamide and myristic fatty acid monoethanolamide.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}".

Further nonionic surfactants as emulsifiers useful in the compositions according to invention are C₁₀-C₂₂-fatty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 25, preferably about 10 and about 20.

Among the non-ionic surfactants mentioned above fatty alcohol ethoxylates and fatty acid alkanolamides and their mixtures at any weight ratio are the most preferred ones.

As a rule any mono alkyl quaternary ammonium surfactants is suitable for the compositions of the present invention as cationic emulsifying surfactant. With the term mono alkyl it is meant that quaternary ammonium surfactant includes only one alkyl chain which has more than 8 or more C atoms.

Preferably at least one mono alkyl quaternary ammonium surfactant is selected from the compounds with the general formula 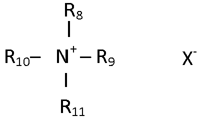 where R₈ is saturated or unsaturated, branched or straight alkyl chain with 8-22 C atoms or

R₁₂CONH(CH₂)ₙ

where R₁₂ is saturated or unsaturated, branched or straight alkyl chain with 7-21 C atoms and n has typical value of 1 - 4 or

R₁₂COO(CH₂)ₙ

where R₁₂ is saturated or unsaturated, branched or straight alkyl chain with 7-21 C atoms and n has typical value of 1 - 4, and
R₉, R₁₀ and R₁₁ are independent from each other lower alkyl chain with 1 to 4 carbon atoms, hydroxyl alky chain with 1 to 4 C atoms, or ethoxy or propoxy group with number of ethoxy or propoxy groups varying in the range of 1 to 4, and X is chloride, bromide or methosulfate.

Suitable cationic surfactants and or conditioning agents are, for example, long-chain quaternary ammonium compounds which can be used alone or in admixture with one another, such as cetyl trimethyl ammonium chloride, myristoyl trimethyl ammonium chloride, behentrimonium chloride, trimethyl cetyl ammonium bromide, stearyl trimethyl ammonium chloride and stearamidopropyltrimethylammonium chloride.

Surfactants are comprised in the aqueous dyeing composition at a total concentration of 0.5 to 20%, preferably 1 to 15% and most preferably 2 - 12.5%, and most preferably 2 to 10% by weight, calculated to the total the dyeing composition.

Dyeing compositions may further comprise one or more polyol. Suitable ones are glycerine, phytantriol, panthenol, ethyleneglycol, polyethyleneglycols, propylene glycols such as 1,2 propylene glycol, 1,3-propylene glycol and polypropylene glycols.

Concentration of one or more polyol is in the range of 0.1 to 15%, preferably 0.25 to 12.5%, more preferably 0.5 to 10% and most preferably 1 to 7.5% by weight calculated to the total of the composition.

The aqueous dyeing composition has a pH between 2 and 11, preferably 6 to 11, more preferably 6.8 to 11, most preferably 8 to 11 and in particular 9 to 10.5.

The oxidizing agents suitable for the oxidizing composition are hydrogen peroxide, urea peroxide, melamin peroxide or perborate salts. The preferred oxidizing agent is hydrogen peroxide, and preferably comprised at a concentration in a range of 1 to 12 % by weight calculated to the total composition.

The pH of the composition comprising at least one oxidizing agent is in the range of 1 to 5, preferably 1.5 to 4 and more preferably 1.5 to 3.

The compositions may additionally comprise an organopolysiloxane wherein at least one silicon atom is linked to an alkylene group having a hetero-atom, in particular a nitrogen atom, with a poly-(N-acyl alkyleneimine) units of the formula 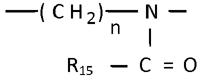 wherein n is a number from 1 to 5 and R₁₅ is hydrogen, a C₁-C₁₂-alkyl or cycloalkyl, aralkyl or aryl group.

Preferred organopolysiloxane polymers are those of the type disclosed in EP-A 640 643, in particular optionally quaternized aminoalkyl, in particular aminopropyl dimethyl polysiloxane/polyethyl oxazoline copolymers of the formula 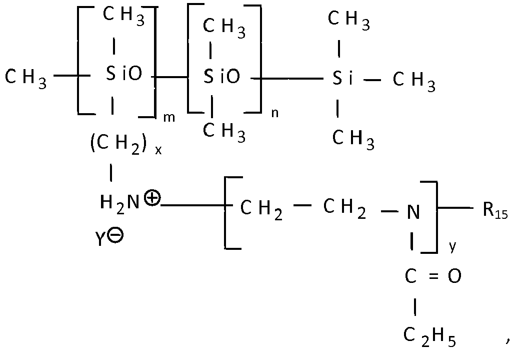 wherein m and n each are numbers from 20 to 10,000, in particular 50 to 7,000, especially 100 to 5,000, x is a number between 1 and 5, preferably 3, and y is a number from 5 to 30, R₁₅ is a C₁-C₁₂-alkyl or aryl group, in particular a methyl, ethyl or benzyl group, and Y⁻ is an anion.

Especially suited are the organopolysiloxanes disclosed under the terms A-1, A-2 and A-3 on pages 12 to 13 of EP-A 640 643. The proportion of graft copolymers in the hair colouring compositions according to the invention ranges from 0.05 % to 5 %, preferably 0.1 % to 2.5 %, in particular 0.5 % to 1.5 % by weight, calculated to the total composition.

Another compound that may be comprised in the dyeing composition is a ceramide type of compounds according to the general formula 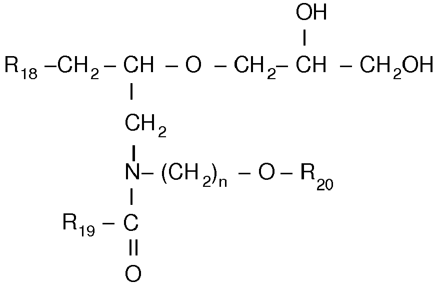 where R₁₈ and R₁₉ are independent from each other alkyl- or alkenyl group with 10 to 22 carbon atoms and R₂₀ is methyl, ethyl, n-propyl or isopropyl group. The concentration of the ceramide type of compound in colouring compositions of the present invention can be in the range of 0.01 to 2 and especially 0.01 to 1% by weight calculated to the total composition.

The dyeing compositions according to the present invention can further comprise one or more ubiquinone of the formula. 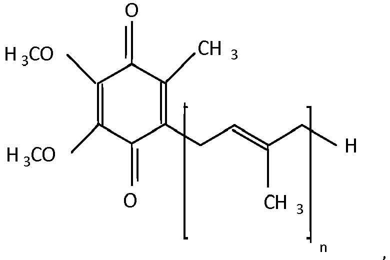 wherein n is a number from 1 to 10. The concentration of ubichinones in the compositions of the present invention can vary between 0.001% and 10% by weight, calculated to the total composition excluding the oxidizing agent.

The dyeing composition may certainly comprise compounds for accelerating (catalysts) the oxidative dyeing keratin fibres such as iodine salts i.e. potassium or sodium iodide and/or dihydroxy acetone.

The aqueous compositions of the present invention may further comprise any compounds found regularly in hair cosmetic compositions such as chelating agents, preservatives, reducing agents, fragrance, acids to adjust the pH and other suitable ones.

Following examples are to illustrate the invention but not to limit it.

### Example 1

| | % by weight |
|---|---|
| Ammonium bicarbonate | 17 |
| Guanidine carbonate | 40 |
| Sodium metasilicate | 8.5 |
| Mineral oil | 30.0 |
| Silica | 4.5 |

The above composition was prepared by mixing all the components in a suitable vessel with each other.

25 g of above composition was mixed with 50 g of an aqueous oxidizing composition comprising hydrogen peroxide at a concentration of 9% by weight and 5% by weight sodium lauryl sulphate and applied onto hair and after leaving 30 min on the hair rinsed off. It was observed that hair was homogeneously bleached.

### Example 2

### Component A:

| | % by weight |
|---|---|
| Ammonium bicarbonate | 15 |
| Guanidine carbonate | 32.5 |
| Mineral oil | 30.0 |
| Silica | 22.5 |

### Component B (emulsion):

| | % by weight |
|---|---|
| Cetearyl alcohol | 20.0 |
| Ceteareth-20 | 10.0 |
| Isopropyl palmitate | 1.0 |
| Dimethicone | 1.0 |
| Polyquaternium-10 | 1.0 |
| Cetrimonium chloride | 1.0 |
| p-Toluenediamine sulphate | 1.25 |
| 1-Naphtthol | 1.25 |
| p-Aminophenol | 1.5 |
| 4-Amino-2-Hydroxytoluene | 1.25 |
| HC Yellow No.2 | 0.25 |
| 1,2 propylene glycol | 3.0 |
| Ammonia (25%) | 10.0 |
| Water | q.s. to 100 |

### Component C (developer):

| | % by weight |
|---|---|
| Hydrogen peroxide | 7.5 |
| Sodium lauryl sulphate | 2.0 |
| Water | q.s. to 100 |

The mixing ratio of the components was 1:2:2, by weight (Parts A:Parts B:Parts C). The composition thus obtained was applied onto hair and after leaving 30 min on the hair, it was rinsed off. It was observed that hair was homogeneously coloured into a reddish colour tone.

## Claims

1. An anhydrous composition for lightening and/or bleaching hair **characterised in that** it comprises at least one alkalizing agent, at least one guanidine salt, at least one second alkaline salt selected from salts of carbonates, bicarbonates, phosphate and hydrogenphosphates with a cation selected from sodium, potassium or ammonium, and at least one oil liquid at 20°C, the oil concentration being in the range of 20 to 60% by weight calculated to the total composition, wherein preferably the composition does not comprise the persalts at a total concentration higher than 10% by weight, preferably 5% by weight and most preferably free of per salts, all values are calculated to the total of the composition.

2. The composition according to claim 1 **characterised in that** at least one guanidine salt is selected form guanidine carbonate, guanidine hydrochloride and guanidine phosphate preferably it is guanidine carbonate and preferably it is comprised at a concentration in the range of 5 to 60%, preferably 10 to 55%, more preferably 15 to 50% and most preferably 15 to 40% by weight calculated to the total of the composition.

3. The composition according to any of the preceding claims characterises in that at least one second alkaline salt selected from sodium carbonate, sodium bicarbonate, trisodium phosphate, disodium hydrogen phosphate, sodium dihydrogen phosphate, potassium carbonate, potassium bicarbonate, tripotassium phosphate, dipotassium hydrogen phosphate, potassium dihydrogen phosphate, ammonium carbonate, ammonium bicarbonate, triammonium phosphate, diammonium hydrogen phosphate and ammonium dihydrogen phosphate, preferably selected from sodium bicarbonate, sodium carbonate, potassium bicarbonate, potassium carbonate, ammonium carbonate and ammonium bicarbonate and more preferably selected from sodium bicarbonate, potassium bicarbonate and ammonium bicarbonate and in particular it is ammonium bicarbonate is the especially preferred alkaline salt which are preferably comprised at a concentration in the range of 5 to 60%, preferably 10 to 55%, more preferably 15 to 50% and most preferably 15 to 40% by weight calculated to the total of the composition.

4. The composition according to any of the preceding claims **characterised in that** at least one guanidine slat and at least one second alkaline salts is comprised in the composition at a weight in the range of 10:1 to 1:5, preferably 5:1 to 1:2, more preferably 3:1 to 1:2 and most preferably 2:1 to 1:1.

5. The composition according to any of the preceding claims **characterised in that** at least one alkalizing agent is sodium metasilicate and is comprised at a concentration in the range of 1 to 20%, preferably 2 to 15% more preferably 2.5 to 12.5% and most preferably 5 to 10% by weight calculated to the total of the composition.

6. The composition according to any of the preceding claims **characterised in that** it comprises an inert carrier material preferably selected from starch, diatomaceous earth, pyrogenic silicium dioxide.

7. The composition according to any of the preceding claims **characterised in that** it comprises one or more oil liquid at 20°C at a concentration in the range of in the range of 25 to 55%, more preferably 25 to 50% and most preferably 30 to 45% by weight calculated to the total of the composition, an preferably selected from synthetic and natural oils wherein the synthetic oils are selected from silicone oils preferably of nonvolatile dimethicones with viscosity of 50 to 350 cSt measured by capillary viscosimeter and at 20°C, fatty acid fatty alcohol esters according to the general structure
R₅₀C(O)R₅₁
wherein R₅₀ is a straight or branched, saturated or unsaturated alkyl with 11 to 21 C atomes and R₅₁ is a straight or branched, saturated or unsaturated alkyl with 1 to 22 C atomes,
fatty alcohol ethers according to general structure
R₃₀OR₄₀
wherein R₃₀ and R₄₀ are same or different, straight or branched, saturated or unsaturated alkyl with 8 to 22 C atoms and natural oils are selected from mineral oil and/or triglycerides such as ricinus oil, soja oil avocado oil, olive oil, almond oil, peach oil, passiflora oil, black cumin oil, borage oils, evening primrose oil, grape seed oil, hempseed oil, kukui nut oil, rosehip oil, safflower oil, walnut oil and wheat germ oil.

8. The composition according to any of the preceding claims **characterised in that** it comprises mineral oil.

9. A process for lightening and/or bleaching human hair wherein the composition according to claims 1 to 8 is mixed with an aqueous composition which has an acidic pH and comprises at least one oxidizing agent and is applied onto hair and after being left on the hair for 1 to 45 min, it is rinsed off from hair, and hair is optionally dried.

10. Kit for hair comprising two or more compositions wherein one of the compositions is the composition according to claims 1 to 8 and the other composition is an aqueous composition comprising at least one oxidizing agent and has an acidic pH.

11. Process for dyeing hair wherein an anhydrous composition comprising at least one guanidine salt and at least one second alkaline salt selected from salts of carbonates, bicarbonates, phosphate and hydrogenphosphates with a cation selected from sodium, potassium or ammonium, and one or more oils liquid at 20°C, the oil concentration being in the range of 20 to 60% by weight calculated to the total composition, is mixed with two aqueous compositions wherein the first composition (Dyeing Composition) is an aqueous composition comprising at least one alkalizing agent and one or more hair dyes and the second composition (Oxidizing Composition) is an aqueous composition comprising at least one oxidizing agent selected from hydrogen peroxide, urea peroxide, melamin peroxide or perborate salts at a concentration in a range of 1 to 12 % by weight calculated to the total oxidizing composition and has a pH in the range of 1 to 5, preferably 1.5 to 4 and more preferably 1.5 to 3, prior to application onto hair and is applied onto hair and is left on the hair for 1 to 45 min and is rinsed off from hair and hair is optionally dried.

12. The process according to claim 11 wherein the alkalizing agent in the dyeing composition is selected from ammonia and compounds according to the general formula
R₁R₂R₃N
wherein R₁, R₂ and R₃ are same or different H, C₁ - C₆ alkyl, C₁ - C₆ monohydroxyalkyl, branched C₃ - C₆ monohydroxyalkyl or C₂ - C₆ polyhydroxyalkyl with the condition that at least one of R₁, R₂ and R₃ is not H and preferably a mono or polyhydroxyalkyl, preferably R₁, R₂ and R₃ are same or different H, C₁ - C₄ alkyl, C₁ - C₄ monohydroxyalkyl, branched C₃ - C₄ monohydroxyalkyl or C₂ - C₄ polyhydroxyalkyl with the condition that at least one of R₁, R₂ and R₃ is not H and preferably a mono or polyhydroxyalkyl.

13. The process according to claims 11 and 12 wherein at least one hair dye is selected from oxidative dye precursors and direct dyes.

14. The process according to claims 11 to 13 wherein the alkalizing agent in the hair dyeing composition is selected from ammonia, monoethanolamine, diethanolamine, triethanolamine, monoethanol methylamine, monoethanoldimethylamine, di-ethanolmethylamine, monoethanolethylamine, monoethanoldiethylamine, diethanolethylamine, monoethanolpropylamine, monoethanoldipropylamine, diethanolpropylamine, monoethanolbutylamine, diethanolbutylamine and amino methyl propanol and their mixtures and comprised at a total concentration between 1 and 35%, preferably 1 and 30%, more preferably 2.5 and 25% and most preferably 2.5 to 20% by weight calculated to the total the dyeing composition.

15. The process according to claims 11 to 14 wherein the dyeing composition is an emulsion and comprises one or more fatty alcohol of the general formula
R₄-OH
wherein R₄ is a linear or branched, saturated or unsaturated alkyl chain with 12 to 22 C atoms and at least one emulsifier selected from anionic, non-ionic, cationic and amphoteric surfactants.

## Patentansprüche

1. Wasserfreie Zusammensetzung zum Aufhellen und/oder Bleichen von Haar, **dadurch gekennzeichnet, dass** sie zumindest ein Alkalisiermittel, zumindest ein Guanidinsalz, zumindest ein zweites alkalisches Salz, ausgewählt aus Salzen von Carbonaten, Bicarbonaten, Phosphat und Hydrogenphosphaten, mit einem Kation, ausgewählt aus Natrium, Kalium oder Ammonium und zumindest ein Öl, das bei 20°C flüssig ist, enthält, wobei die Ölkonzentration im Bereich von 20 bis 60 Gew.% ist, berechnet auf die gesamte Zusammensetzung, worin bevorzugt die Zusammensetzung nicht die Persalze bei einer Gesamtkonzentration von mehr als 10 Gew.%, bevorzugt 5 Gew.% enthält und am meisten bevorzugt frei von Salzen ist, wobei alle Werte in Bezug auf die gesamte Zusammensetzung berechnet sind.

2. Zusammensetzung gemäß Anspruch 1, **dadurch gekennzeichnet, dass** das zumindest eine Guanidinsalz ausgewählt ist aus Guanidincarbonat, Guanidinhydrochlorid und Guanidinphosphat, bevorzugt Guanidincarbonat ist und bevorzugt bei einer Konzentration in dem Bereich von 5 bis 60 Gew.%, bevorzugt 10 bis 55 Gew.%, mehr bevorzugt 15 bis 50 Gew.% und am meisten bevorzugt 15 bis 40 Gew.%, bezogen auf das Gesamte der Zusammensetzung, enthalten ist.

3. Zusammensetzung gemäß einem der vorhergehenden Ansprüche, **dadurch gekennzeichnet, dass** zumindest ein zweites Alkalisalz, ausgewählt aus Natriumcarbonat, Natriumbicarbonat, Trinatriumphosphat, Dinatriumhydrogenphosphat, Natriumdihydrogenphosphat, Kaliumcarbonat, Kaliumbicarbonat, Trikaliumphosphat, Dikaliumhydrogenphosphat, Kaliumdihydrogenphosphat, Ammoniumcarbonat, Ammoniumbicarbonat, Triammoniumphosphat, Diammoniumhydrogenphosphat und Ammoniumdihydrogenphosphat, bevorzugt ausgewählt aus Natriumbicarbonat, Natriumcarbonat, Kaliumbicarbonat, Kaliumcarbonat, Ammoniumcarbonat und Ammoniumbicarbonat und mehr bevorzugt ausgewählt aus Natriumbicarbonat, Kaliumbicarbonat und Ammoniumbicarbonat und Ammoniumbicarbonat das besonders bevorzugtes Alkalisalz ist, die bevorzugt bei einer Konzentration im Bereich von 5 bis 60 Gew.%, bevorzugt 10 bis 55 Gew.%, mehr bevorzugt 15 bis 50 Gew.% und am meisten bevorzugt 15 bis 40 Gew.%, berechnet auf das Gesamte der Zusammensetzung, enthalten sind.

4. Zusammensetzung gemäß einem der vorhergehenden Ansprüche, **dadurch gekennzeichnet, dass** zumindest ein Guanidinsalz und zumindest ein zweites Alkalisalz in der Zusammensetzung bei einem Gewicht im Bereich von 10:1 bis 1:5, bevorzugt 5:1 bis 1:2, mehr bevorzugt 3:1 bis 1:2 und am meisten bevorzugt 2:1 bis 1:1 enthalten sind.

5. Zusammensetzung gemäß einem der vorhergehenden Ansprüche, **dadurch gekennzeichnet, dass** zumindest ein Alkalisiermittel Natriummetasilikat ist und in einer Konzentration im Bereich von 1 bis 20 Gew.%, bevorzugt 2 bis 15 Gew.%, mehr bevorzugt 2,5 bis 12,5 Gew.% und am meisten bevorzugt 5 bis 10 Gew.%, berechnet auf das Gesamte der Zusammensetzung, enthalten ist.

6. Zusammensetzung gemäß einem der vorhergehenden Ansprüche, **dadurch gekennzeichnet, dass** sie ein inertes Trägermaterial enthält, bevorzugt ausgewählt aus Stärke, Diatomeenerde, pyrogenem Siliciumdioxid.

7. Zusammensetzung gemäß einem der vorhergehenden Ansprüche, **dadurch gekennzeichnet, dass** sie eine oder mehrere Öle, die bei 20°C flüssig sind, bei einer Konzentration im Bereich von 25 bis 55 Gew.%, mehr bevorzugt 25 bis 50 Gew.% und am meisten bevorzugt 30 bis 45 Gew.% enthält, berechnet auf das Gesamte der Zusammensetzung, und bevorzugt ausgewählt aus synthetischen und natürlichen Ölen, worin die synthetischen Öle ausgewählt sind aus Silikonölen, bevorzugt von nichtflüchtigen Dimethiconen mit einer Viskosität von 50 bis 350 cSt, gemessen durch Kapillarviskosimeter und bei 20°C, Fettsäurefettalkoholestern gemäß der allgemeinen Struktur
R₅₀C(O)R₅₁
worin R₅₀ ein geradkettiges oder verzweigtes, gesättigtes oder ungesättigtes Alkyl mit 11 bis 21 C-Atomen und R₅₁ geradkettiges oder verzweigtes, gesättigtes oder ungesättigtes Alkyl mit 1 bis 22 C-Atomen ist, Fettalkoholethern gemäß der allgemeinen Struktur
R₃₀OR₄₀
worin und R₄₀ gleich oder verschieden sind und geradkettiges oder verzweigtes, gesättigtes oder ungesättigtes Alkyl mit 8 bis 22 C-Atomen und die natürlichen öle ausgewählt sind aus Mineralöl und/oder Triglyceriden, wie Rizinusöl, Sojaöl, Avocadoöl, Olivenöl, Mandelöl, Pfirsichöl, Passifloraöl, Schwarzkümmelöl, Boretschöl, Nachtkerzenöl, Traubenkernöl, Hanföl, Kukui-Nussöl, Hagebuttenöl, Distelöl, Walnussöl und Weizenkeimöl.

8. Zusammensetzung gemäß einem der vorhergehenden Ansprüche, **dadurch gekennzeichnet, dass** sie Mineralöl enthält.

9. Verfahren zum Aufhellen und/oder Bleichen von menschlichem Haar, worin die Zusammensetzung gemäß den Ansprüchen 1 bis 8 mit einer wässrigen Zusammensetzung gemischt wird, die einen sauren pH hat und zumindest ein Oxidationsmittel enthält und auf das Haar aufgetragen und nach Verbleiben auf dem Haar für 1 bis 45 Minuten von dem Haar abgespült und das Haar wahlweise getrocknet wird.

10. Kit für Haar, enthaltend zwei oder mehrere Zusammensetzungen, worin eine der Zusammensetzungen die Zusammensetzung gemäß den Ansprüchen 1 bis 8 ist und die andere Zusammensetzung eine wässrige Zusammensetzung ist, die zumindest ein Oxidationsmittel enthält und einen sauren pH hat.

11. Verfahren zum Färben von Haar, worin eine wasserfreie Zusammensetzung, enthaltend zumindest ein Guanidinsalz und zumindest ein zweites Alkalisalz, ausgewählt aus Salzen von Carbonaten, Bicarbonaten, Phosphat und Hydrodenphosphaten, mit einem Kation, ausgewählt aus Natrium, Kalium oder Ammonium, und ein oder mehrere Öle, die bei 20°C flüssig sind, wobei die Ölkonzentration im Bereich von 20 bis 60 Gew.% ist, berechnet auf die gesamte Zusammensetzung, mit zwei wässrigen Zusammensetzungen vermischt wird, worin die erste Zusammensetzung (Färbezusammensetzung) eine wässrige Zusammensetzung ist, enthaltend zumindest ein Alkalisiermittel und einen oder mehrere Farbstoffe und die zweite Zusammensetzung (Oxidationszusammensetzung) eine wässrige Zusammensetzung ist, enthaltend zumindest ein Oxidationsmittel, ausgewählt aus Wasserstoffperoxid, Harnstoffperoxid, Melaminperoxid oder Perboratsalzen bei einer Konzentration in einem Bereich von 1 bis 12 Gew.-%, berechnet auf die gesamte oxidierende Zusammensetzung und einen pH im Bereich von 1 bis 5, bevorzugt 1,5 bis 4 und mehr bevorzugt 1,5 bis 3 hat, vor der Auftragung auf das Haar und auf das Haar aufgetragen und auf dem Har 1 bis 45 Minuten belassen und vom Haar abgespült und das Haar wahlweise getrocknet wird.

12. Verfahren gemäß Anspruch 11, worin das Alkalisiermittel in der Färbezusammensetzung ausgewählt ist aus Ammoniak und Verbindungen gemäß der allgemeinen Formel
R₁R₂R₃N
worin R₁, R₂ und R₃ gleich oder verschieden sind und H, C₁₋₆-Alkyl, C₁₋₆-Monohydroxyalkyl, verzweigtes C₃₋₆-Monohydroxyalkyl oder C₂₋₆-Polyhydroxyalkyl sind, mit der Bedingung, dass zumindest eines von R₁, R₂ und R₃ nicht H ist, und bevorzugt ein Mono- oder Polyhydroxyalkyl sind, worin bevorzugt R₁, R₂ und R₃ gleich oder verschieden sind und H, C₁₋₄-Alkyl, C₁₋₄-Monohydroxyalkyl, verzweigtes C₃₋₄-Monohydroxyalkyl oder C₂₋₄-Polyhydroxyalkyl sind, mit der Bedingung, dass zumindest eines von R₁, R₂ und R₃ nicht H ist und bevorzugt ein Mono- oder Polyhydroxyalkyl ist.

13. Verfahren gemäß Ansprüchen 11 und 12, worin zumindest ein Haarfarbstoff ausgewählt ist aus oxidativen Farbstoffvorläufern und direktziehenden Farbstoffen.

14. Verfahren gemäß Ansprüchen 11 bis 13, worin das Alkalisiermittel in der Haarfärbezusammensetzung ausgewählt ist aus Ammoniak, Monoethanolamin, Diethanolamin, Triethanolamin, Monoethanolmethylamin, Monoethanoldimethylamin, Diethanolmethylamin, Monoethanolethylamin, Monoethanoldiethylamin, Diethanolethylamin, Monoethanolpropylamin, Monoethanoldipropylamin, Diethanolpropylamin, Monoethanolbutylamin, Diethanolbutylamin und Aminomethylpropanol und deren Mischungen und bei einer Gesamtkonzentration zwischen 1 und 35 Gew.%, bevorzugt 1 bis 30 Gew%., mehr bevorzugt 2,5 bis 25 Gew.% und am meisten bevorzugt 2,5 bis 20 Gew.% enthalten ist, berechnet auf das Gesamte der Färbezusammensetzung.

15. Verfahren gemäß den Ansprüchen 11 bis 14, worin die Färbezusammensetzung eine Emulsion ist und einen oder mehrere Fettalkohole der allgemeinen Formel
R₄-OH
worin R₄ eine lineare oder verzweigte, gesättigte oder ungesättigte Alkylkette mit 12 bis 22 C-Atomen ist, und zumindest einen Emulgator enthält, ausgewählt aus anionischen, nicht-ionischen, kationischen und amphoteren Tensiden.

## Revendications

1. Composition anhydre pour l'éclaircissement et/ou le blanchiment des cheveux, **caractérisée en ce qu'**elle comprend au moins un agent alcalinisant, au moins un sel de guanidine, au moins un second sel alcalin choisi parmi des sels de carbonates, de bicarbonates, de phosphates et d'hydrogénophosphates avec un cation choisi parmi le sodium, le potassium ou l'ammonium, et au moins une huile liquide à 20°C, la concentration de l'huile étant située dans la plage de 20 à 60% en poids calculée par rapport à la composition totale, dans laquelle de préférence la composition ne comprend pas les persels à une concentration totale supérieure à 10% en poids, de préférence 5 % en poids et de façon préférée entre toutes est dépourvue de persels, toutes les valeurs étant calculées par rapport au total de la composition.

2. Composition selon la revendication 1, **caractérisée en ce qu'**au moins un sel de guanidine est choisi parmi le carbonate de guanidine, le chlorhydrate de guanidine et le phosphate de guanidine, de préférence est le carbonate de guanidine et de préférence est compris à une concentration située dans la plage de 5 à 60 %, de préférence de 10 à 55 %, plus de préférence de 15 à 50 % et le plus de préférence de 15 à 40 % en poids calculée par rapport au total de la composition.

3. Composition selon l'une quelconque des revendications précédentes, **caractérisée en ce qu'**au moins un second sel alcalin choisi parmi le carbonate de sodium, le bicarbonate de sodium, le phosphate trisodique, l'hydrogénophosphate disodique, le dihydrogénophosphate de sodium, le carbonate de potassium, le bicarbonate de potassium, le phosphate tripotassique, l'hydrogénophosphate dipotassique, le dihydrogénophosphate de potassium, le carbonate d'ammonium, le bicarbonate d'ammonium, le phosphate de triammonium, l'hydrogénophosphate de diammonium et le dihydrogénophosphate d'ammonium, de préférence choisi parmi le bicarbonate de sodium, le carbonate de sodium, le bicarbonate de potassium, le carbonate de potassium, le carbonate d'ammonium et le bicarbonate d'ammonium et le plus de préférence choisi parmi le bicarbonate de sodium, le bicarbonate de potassium et le bicarbonate d'ammonium et en particulier le bicarbonate d'ammonium est le sel alcalin spécialement préféré qui est compris de préférence à une concentration située dans la plage de 5 à 60 %, de préférence de 10 à 55 %, plus de préférence de 15 à 50 % et le plus de préférence de 15 à 40 % en poids calculée par rapport au total de la composition.

4. Composition selon l'une quelconque des revendications précédentes, **caractérisée en ce qu'**au moins un sel de guanidine et au moins un second sel alcalin sont compris dans la composition a un poids situé dans la plage de 10:1 à 1:5, de préférence de 5:1 à 1:2, plus de préférence de 3:1 à 1:2 et le plus de préférence de 2:1 à 1:1.

5. Composition selon l'une quelconque des revendications précédentes, **caractérisée en ce que** le au moins un agent alcalinisant est le métasilicate de sodium et est compris à une concentration située dans la plage de 1 à 20 %, de préférence de 2 à 15 %, plus de préférence de 2,5 à 12,5% et le plus de préférence de 5 à 10% en poids calculée par rapport au total de la composition.

6. Composition selon l'une quelconque des revendications précédentes, **caractérisée en ce qu'**elle comprend un matériau support inerte choisi de préférence parmi l'amidon, la terre de diatomée, la silice pyrogénée.

7. Composition selon l'une quelconque des revendications précédentes, **caractérisée en ce qu'**elle comprend une ou plusieurs huiles liquides à 20°C à une concentration située dans la plage de 25 à 55%, plus de préférence de 25 à 50% et le plus de préférence de 30 à 45 % en poids calculée par rapport au total de la composition, une choisie de préférence parmi des huiles synthétiques et naturelles, dans laquelle les huiles synthétiques sont choisies parmi des huiles de silicone, de préférence des diméthicones non volatiles, avec une viscosité de 50 à 350 cSt mesurée par un viscosimètre capillaire et à 20°C, des esters d'alcools gras d'acides gras selon la structure générale
R₅₀C(O)R₅₁
dans laquelle R₅₀ représente un alkyle saturé ou insaturé, linéaire ou ramifié, avec 11 à 21 atomes de C et R₅₁ représente un alkyle saturé ou insaturé, linéaire ou ramifié, avec 1 à 22 atomes de C, des éthers d'alcools gras selon la structure générale
R₃₀OR₄₀
dans laquelle R₃₀ et R₄₀, identiques ou différents, représentent un groupe alkyle saturé ou insaturé, linéaire ou ramifié, avec 8 à 22 atomes de C, et les huiles naturelles sont choisies parmi une huile minérale et/ou des triglycérides tels que l'huile de ricin, l'huile de soja, l'huile d'avocat, l'huile d'olive, l'huile d'amande, l'huile de pêche, l'huile de passiflore, l'huile de cumin noir, les huiles de bourrache, l'huile d'onagre, l'huile de pépins de raisin, l'huile de chanvre, l'huile de noix de Kukui, l'huile d'églantier, l'huile de carthame, l'huile de noix et l'huile de germe de blé.

8. Composition selon l'une quelconque des revendications précédentes, **caractérisée en ce qu'**elle comprend une huile minérale.

9. Procédé pour l'éclaircissement et/ou le blanchiment des cheveux humains, dans lequel la composition selon les revendications 1 à 8 est mélangée avec une composition aqueuse qui a un pH acide et comprend au moins un agent oxydant et est appliquée sur les cheveux et, après avoir été laissée sur les cheveux pendant 1 à 45 min, est rincée des cheveux, et les cheveux sont éventuellement séchés.

10. Kit pour les cheveux comprenant deux compositions ou plus, dans lequel l'une des compositions est la composition selon les revendications 1 à 8 et l'autre composition est une composition aqueuse comprenant au moins un agent oxydant et a un pH acide.

11. Procédé pour la coloration des cheveux, dans lequel une composition anhydre comprenant au moins un sel de guanidine et au moins un second sel alcalin choisi parmi des sels de carbonates, de bicarbonates, de phosphates et d'hydrogénophosphates avec un cation choisi parmi le sodium, le potassium ou l'ammonium, et une ou plusieurs huiles liquides à 20°C, la concentration des huiles étant située dans la plage de 20 à 60% en poids calculée par rapport à la composition totale, est mélangée avec deux compositions aqueuses, dans lequel la première composition (composition colorante) est une composition aqueuse comprenant au moins un agent alcalinisant et un ou plusieurs colorants pour les cheveux, et la seconde composition (composition oxydante) est une composition aqueuse comprenant au moins un agent oxydant choisi parmi le peroxyde d'hydrogène, le peroxyde d'urée, le peroxyde de mélamine ou des sels de perborates à une concentration située dans la plage de 1 à 12 % en poids calculée par rapport à la composition oxydante totale, et a un pH situé dans la plage de 1 à 5, de préférence de 1,5 à 4 et plus de préférence de 1,5 à 3, avant l'application sur les cheveux, et est appliquée sur les cheveux et est laissée sur les cheveux pendant 1 à 45 min et est rincée des cheveux, et les cheveux sont éventuellement séchés.

12. Procédé selon la revendication 11 dans lequel l'agent alcalinisant dans la composition colorante est choisi parmi l'ammoniac et des composés selon la formule générale
R₁R₂R₃N
dans lequel R₁, R₂ et R₃, identiques ou différents, représentent H, un groupe alkyle en C₁ à C₆, monohydroxyalkyle en C₁ à C₆, monohydroxyalkyle en C₃ à C₆ ramifié ou polyhydroxyalkyle en C₂ à C₆, à condition qu'au moins l'un de R₁, R₂ et R₃ ne représente pas H et de préférence un groupe mono- ou polyhydroxyalkyle, de préférence R₁, R₂ et R₃, identiques ou différents, représentent H, un groupe alkyle en C₁ à C₄, monohydroxyalkyle en C₁ à C₄, monohydroxyalkyle en C₃ à C₄ ramifié ou polyhydroxyalkyle en C₂ à C₄, à condition qu'au moins l'un de R₁, R₂ et R₃ ne représente pas H, et de préférence un groupe mono- ou polyhydroxyalkyle.

13. Procédé selon les revendications 11 et 12, dans lequel au moins un colorant pour les cheveux est choisi parmi des précurseurs de colorants oxydants et des colorants directs.

14. Procédé selon les revendications 11 à 13, dans lequel l'agent alcalinisant dans la composition colorante pour les cheveux est choisi parmi l'ammoniac, la monoéthanolamine, la diéthanolamine, la triéthanolamine, la monoéthanol-méthylamine, la monoéthanol-diméthylamine, la diéthanol-méthylamine, la monoéthanol-éthylamine, la monoéthanol-diéthylamine, la diéthanol-éthylamine, la monoéthanol-propylamine, la monoéthanol-dipropylamine, la diéthanol-propylamine, la monoéthanol-butylamine, la diéthanol-butylamine et l'aminométhylpropanol et leurs mélanges, et est compris à une concentration totale située entre 1 et 35 %, de préférence entre 1 et 30%, plus de préférence entre 2,5 et 25% est le plus de préférence entre 2,5 et 20% en poids calculée par rapport au total de la composition colorante.

15. Procédé selon les revendications 11 à 14, dans lequel la composition colorante est une émulsion et comprend un ou plusieurs alcools gras de la formule générale
R₄-OH
dans lequel R₄ représente une chaîne alkyle saturée ou insaturée, linéaire ou ramifiée, avec 12 à 22 atomes de C et au moins un émulsifiant choisi parmi des tensioactifs anioniques, non ioniques, cationiques et amphotères.
